(19) Europäisches Patentamt
European Patent Office
Office européen des brevets

(11) EP 4 494 613 A1

(12) **EUROPEAN PATENT APPLICATION**
published in accordance with Art. 153(4) EPC

(43) Date of publication:
**22.01.2025 Bulletin 2025/04**

(21) Application number: **23770795.5**

(22) Date of filing: **14.03.2023**

(51) International Patent Classification (IPC):
***A61F 9/007*** *(2006.01)* ***A61L 27/14*** *(2006.01)*
***A61L 27/50*** *(2006.01)*

(52) Cooperative Patent Classification (CPC):
**A61F 9/007; A61L 27/14; A61L 27/50**

(86) International application number:
**PCT/JP2023/009870**

(87) International publication number:
**WO 2023/176840 (21.09.2023 Gazette 2023/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.03.2022 JP 2022039953**

(71) Applicant: **National University Corporation Okayama University**
**Kita-ku,**
**Okayama-shi,**
**Okayama 700-8530 (JP)**

(72) Inventors:
- **UCHIDA, Tetsuya**
**Okayama-shi, Okayama 700-8530 (JP)**
- **MATSUO, Toshihiko**
**Okayama-shi, Okayama 700-8530 (JP)**

(74) Representative: **Müller-Boré & Partner Patentanwälte PartG mbB**
**Friedenheimer Brücke 21**
**80639 München (DE)**

(54) **ARTIFICIAL RETINA AND METHOD FOR PRODUCING SAME**

(57) There is provided an artificial retina in which an organic dye compound is fixed onto a thin sheet, wherein the organic dye compound induces a receptor potential responding to photostimulation; the thin sheet is formed by connecting microfibers having an average fiber diameter of 2 to 1,000 nm to each other to form a porous structure; and the porous structure makes the artificial retina liquid-permeable. Thus, there is provided an artificial retina that can induce a receptor potential responding to photostimulation with high sensitivity and is liquid-permeable and therefore has improved biocompatibility.

[FIG. 4]

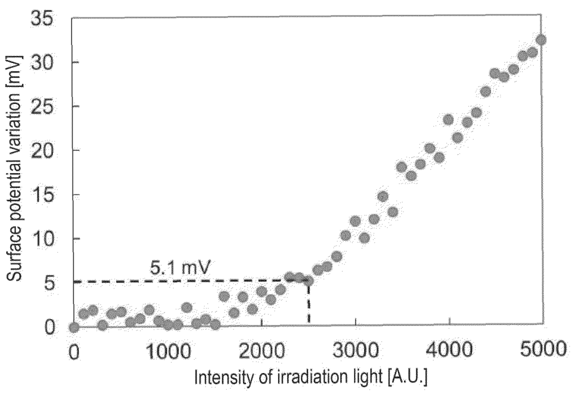




Processed by Luminess, 75001 PARIS (FR)

## Description

### Technical Field

The present invention relates to an artificial retina in which an organic dye compound that induces a receptor potential responding to photostimulation in optic nerve is fixed, and a production method therefor.

### Background Art

It is known that some organic dye compounds induce a receptor potential responding to photostimulation in optic nerve, particularly retinal neuronal cells constituting optic nerve. There have been investigations for alternative retinal materials using such an organic dye compound.

Patent Literature 1 has described an agent for inducing receptor potential containing an organic dye compound which induces a receptor potential responding to photostimulation in optic nerve. According to this, it is described that the agent for inducing receptor potential is very useful as a substituent material for visual-related substances in substituent materials for the retina such as an artificial retina for alleviating or eliminating visual disturbance caused by a retinal disorder associated with injury or sickness including visual field constriction, decreased vision and nyctalopia, and color anomaly caused by chemical addiction, neural disturbance of visual center, retinal disease and lack of a particular retinal cone.

Patent Literature 2 describes a method for producing an artificial retina in which an organic dye compound that induces a receptor potential responding to photostimulation is fixed onto a polymer sheet substrate, comprising a bonding step of immersing the substrate in a solution containing the organic dye compound to chemically bond the organic dye compound to the substrate; a first washing step of washing with water the substrate to which the organic dye compound has been chemically bonded; and a second washing step of, after the first washing step, washing with an organic solvent the substrate to which the organic dye compound has been chemically bonded. The Reference describes that the method allows for providing an artificial retina excellent in mechanical properties such as breaking elongation, which is favorably biocompatible and can induce a receptor potential responding to photostimulation with high sensitivity.

However, the artificial retina described in these Patent Literatures are, however, organic dye compounds fixed onto a polyethylene film or the like, and are not permeable to a fluid such as a body fluid and blood.

### Citation List

#### Patent Literature

Patent Literature 1: JP 2004-121292 A
Patent Literature 2: WO 2015/152233 A1

### Summary of Invention

#### Technical Problem

To solve the above problems, an objective of the present invention is to provide an artificial retina that can induce a receptor potential responding to photostimulation with high sensitivity and is liquid-permeable and therefore has improved biocompatibility.

#### Solution to Problem

The above problems can be solved by providing an artificial retina in which an organic dye compound is fixed onto a thin sheet, wherein

the organic dye compound induces a receptor potential responding to photostimulation;
the thin sheet is formed by connecting microfibers having an average fiber diameter of 2 to 1,000 nm to each other to form a porous structure; and
the porous structure makes the artificial retina liquid-permeable.

Here, it is preferable that the organic dye compound is fixed onto the microfibers in the thin sheet via a covalent

bond, and that the microfibers are biocompatible.

The above problems can be also solved by providing a method for producing the artificial retina, comprising

diamine-modifying the microfibers having carboxy groups;
reacting the organic dye compound with the diamine-modified microfibers to fix the organic dye compound to the microfibers; and then
producing the thin sheet such that the microfibers are connected to each other to form a porous structure.

The above problems can be also solved by providing a method for producing the artificial retina, comprising

producing the thin sheet such that the microfibers are connected to each other to form a porous structure; then
diamine-modifying the microfibers which have carboxy groups and constitute the thin sheet; and
reacting the organic dye compound with the diamine-modified microfibers constituting the thin sheet to fix the organic dye compound to the microfibers.

Advantageous Effects of Invention

In accordance with the present invention, there can be provided an artificial retina that can induce a receptor potential responding to photostimulation with high sensitivity and is liquid-permeable and therefore has improved biocompatibility.

Brief Description of Drawings

FIG. 1 is a photo of the TOCN porous material in Example 1.
FIG. 2 is a photo of the dye-fixed thin sheet in Example 1.
FIG. 3 is an EDX spectrum of the dye-fixed thin sheet in Example 1.
FIG. 4 is a graph showing surface potential variation of the dye-fixed thin sheet in Example 1.
FIG. 5 is an IR spectrum of the TOCN thin sheet and the TOCN-COOH thin sheet in Example 2.
FIG. 6 is a photo of the dye-fixed TOCN-COOH thin sheet in Example 2.
FIG. 7 is an EDX spectrum of the dye-fixed TOCN-COOH thin sheet in Example 2.
FIG. 8 is a graph showing surface potential variation of the dye-fixed TOCN-COOH thin sheet in Example 2.
FIG. 9 shows the results of ultraviolet-visible spectrometry for the dye-fixed TOCN-COOH thin sheet in Example 2 and the conventional dye-fixed thin film.

Description of Embodiments

An artificial retina of the present invention is an artificial retina in which an organic dye compound is fixed onto a thin sheet, wherein the organic dye compound induces a receptor potential responding to photostimulation; the thin sheet is formed by connecting microfibers having an average fiber diameter of 2 to 1,000 nm to each other to form a porous structure; and the porous structure makes the artificial retina liquid-permeable. It is advantageously liquid-permeable and has improved biocompatibility because the thin sheet has a porous structure in which microfibers having an average fiber diameter of 2 to 1,000 nm are connected to each other. In the present invention, liquid permeability means that liquid that has permeated from the surface of the artificial retina passes through to the back surface.

There are no particular restrictions to an organic dye compound used in the present invention as long as it can induce a receptor potential responding to photostimulation; examples include acridine dyes, azaannulene dyes, azo dyes, anthraquinone dyes, indigo dyes, indanthrene dyes, oxazine dyes, xanthene dyes, coumarin dyes, dioxadine dyes, thiazine dyes, thioindigo dyes, tetraporphyrazine dyes, triphenylmethane dyes, triphenothiazine dyes, naphthoquinone dyes, phthalocyanine dyes, benzoquinone dyes, benzopyrane dyes, benzofuranone dyes, polymethine dyes, porphyrin dyes and rhodamine dyes. Among these, a polymethine type organic dye compound can be suitably used. Specific suitable examples of such a polymethine type organic dye compound include those represented by Chemical Formulas 1 to 17 in Patent No. 5090431 or that represented by Chemical Formula 2 (NK-10714) in JP 2020-146296A.

The thin sheet in the artificial retina of the present invention has a porous structure formed by connecting microfibers having an average fiber diameter of 2 to 1,000 nm to each other. A porous structure herein means spaces formed by connecting microfibers to each other. There are no particular limitations to the porous structure, as long as pores are formed that allow a liquid that has permeated from the surface of the thin sheet to pass through to the back surface. A porosity of the porous structure is preferably, but not limited to, 5 to 99.9%. The porosity is more preferably 10% or more,

further preferably 20% or more, particularly preferably 50% or more. Meanwhile, the porosity is more preferably 99% or less. The porosity can be determined by the method described in Examples below.

Microfibers used in the present invention can include, but not limited to, natural fibers such as cellulose, chitin and chitosan; and synthetic fibers including polyolefin fibers such as polyethylene and polypropylene, polyester fibers such as polyethylene terephthalate and polybutylene terephthalate, polyamide fibers such as Nylons, and polyvinyl alcohol fibers such as vinylon. Among these, cellulose nanofibers (CNF), which are representative of natural fibers, have the advantage of being highly biocompatible with cells and the like due to their good flexibility, and also advantageously have excellent chemical and physical stability in vivo. It is an important feature that a material used in the artificial retina of the present invention is biocompatible. It is, therefore, preferable that the microfibers are biocompatible. Here, biocompatibility means that they can be used without deteriorating cells and the like. In this light, the microfibers are preferably made of natural fibers, more preferably made of at least one selected from the group consisting of cellulose, chitin and chitosan.

An average fiber diameter of the microfibers is 2 to 1,000 nm. If an average fiber diameter is less than 2 nm, strength may be insufficient when a thin sheet is produced such that the microfibers are connected to each other to form a porous structure. Thus, an average fiber diameter is preferably 3 nm or more, more preferably 4 nm or more. Meanwhile, if an average fiber diameter is more than 1,000 nm, it may be difficult to form a porous structure which is liquid-permeable. Thus, an average fiber diameter is preferably 800 nm or less, more preferably 600 nm or less, further preferably 500 nm or less, particularly preferably 400 nm or less.

The present invention provides an artificial retina onto which an organic dye compound which induces receptor potential responding to photostimulation is fixed. Herein, fixing the organic dye compound means that the organic dye compound is chemically fixed onto the microfibers in the thin sheet. A preferable embodiment is that the organic dye compound is fixed onto the microfibers via a covalent bond. Examples of the covalent bond can be, but not limited to, an amide bond, an ester bond, an ether bond, a urethane bond or the like, and among these, an amide bond is preferred. Since the artificial retina of the present invention has a porous structure in which the microfibers are connected to each other, it is possible to fix a larger amount of the organic dye compound than conventional one, and the effect of improving a resolution can be expected when used as an artificial retina.

In an artificial retina of the present invention, a contact angle of water to the surface of the artificial retina is preferably 50 to 90 °. With a water contact angle being within such a range, biocompatibility is advantageously improved. A water contact angle is more preferably 60 ° or higher, further preferably 70 ° or higher, particularly preferably 75 ° or higher. Meanwhile, a water contact angle is more preferably 85 ° or less, further preferably 80° or less.

A thickness of the artificial retina of the present invention is preferably 5 to 100 μm. If the thickness is less than 5 μm, strength may decrease, so the thickness is more preferably 10 μm or more. Meanwhile, if the thickness exceeds 100 μm, it may become difficult to insert the artificial retina into the eyeball, so a thickness is more preferably 80 μm or less, further preferably 60 μm or less.

There are no particular restrictions to a method for producing the artificial retina of the present invention. A preferable embodiment is a method for producing an artificial retina comprising diamine-modifying the microfibers having carboxy groups; reacting the organic dye compound with the diamine-modified microfibers to fix the organic dye compound to the microfibers; and then producing the thin sheet such that the microfibers are connected to each other to form a porous structure (hereinafter, sometimes referred to as "the first production method"). Another preferable embodiment is a method for producing an artificial retina comprising producing the thin sheet such that the microfibers are connected to each other to form a porous structure; then diamine-modifying the microfibers which have carboxy groups and constitute the thin sheet; and reacting the organic dye compound with the diamine-modified microfibers constituting the thin sheet to fix the organic dye compound to the microfibers (hereinafter, sometimes referred to as "the second production method").

The first production method will be described. In the first production method, it is preferable to carry out diamine modification on the microfibers having carboxy groups. This allows the diamine-modified microfibers to easily react with the organic dye compound. There are no particular restrictions to the method for diamine-modifying. For example, a method comprising reacting the microfibers having carboxy groups with ethylenediamine using DCC (N,N-dicyclohexylcarbodiimide) is preferably employed. Thus, the carboxy group is subject to dehydration condensation with an amine to form an amide bond, so that the diamine-modified microfibers obtained have an amino group.

It is also a preferable embodiment to acid-treat the microfibers having carboxy groups before diamine modification. When the carboxy groups of the microfibers are salts such as COONa type, the dehydration condensation reaction with the amine may not proceed sufficiently. Therefore, it is a preferable embodiment to convert the carboxy groups of the microfibers to COOH type by acid treatment. A preferable acid treatment is immersing the microfibers having carboxy groups in an acid such as hydrochloric acid, sulfuric acid and nitric acid.

Next, it is preferable to react the organic dye compound with the diamine-modified microfibers for fixation. There are no particular restrictions to a method for reacting the organic dye compound; for example, when the organic dye compound has a carboxy group, the reaction is conducted in the presence of DCC as in the above diamine modification. Thus, an amino group of the diamine-modified microfibers is subjected to dehydration-condensation with a carboxy group

of the organic dye compound to form an amide bond, so that the organic dye compound can be fixed onto the diamine-modified microfibers.

Examples of a solvent which can be used in the reaction include, but not limited to, halogenated hydrocarbon solvents such as chlorobenzene, dichlorobenzene, trichlorobenzene, dichloroethane, trichloroethane, dichloromethane, chloroform and carbon tetrachloride; saturated aliphatic hydrocarbon solvents such as pentane, hexane, heptane, octane and cyclohexane; aromatic hydrocarbon solvents such as benzene, toluene and xylene; ether solvents such as dimethyl ether, ethyl methyl ether, diethyl ether, tetrahydrofuran and 1,4-dioxane; nitrile solvents such as acetonitrile, propionitrile and benzonitrile; and aprotic polar solvents such as dimethyl sulfoxide, N,N-dimethylformamide and N-methylpyrrolidone. Among these, at least one organic solvent selected from the group consisting of halogenated hydrocarbon solvents, saturated aliphatic hydrocarbon solvents and aromatic hydrocarbon solvents is suitably used, and halogenated hydrocarbon solvents are more suitably used.

An artificial retina of the present invention can be provided by producing a thin sheet such that such that the microfibers on which the organic dye compound has been fixed are connected to each other to form a porous structure. There are no particular restrictions to a method for producing the thin sheet, and a method wherein a dispersion containing the microfibers is made into a sheet, which is then dried is preferably used. In terms of making a sheet, a dispersion containing the microfibers can be poured into a vessel to form a sheet; and a dispersion containing the microfibers can be coated on a substrate to form a sheet. After forming a sheet, a dispersion medium is removed by drying to provide a thin sheet. Drying can be, but not limited to, natural drying, drying by heating and freeze drying as appropriate. The dispersion medium is preferably water, and an organic solvent can be contained in such an amount that it does not dissolve the fixed organic dye compound. The organic solvent may be those used in the reaction.

Concentrations of the microfibers in the microfiber-containing dispersion is preferably, but not limited to, 0.01 to 5 % by weight. If the microfiber concentration is less than 0.01 % by weight, there is possibility that the film forming cannot be conducted. Thus, the concentration is more preferably 0.05 % by weight or more, further preferably 0.1 % by weight or more. Meanwhile, if the microfiber concentration is more than 5 % by weight, uneven density may occur, leading to insufficient liquid permeability. Thus, the concentration is more preferably 3 % by weight or less, further preferably 1 % by weight or less, particularly preferably 0.8 % by weight or less.

The dispersion containing the microfibers can contain an adhesive polymer in the light of enhancing bonding of the microfibers to each other. Examples of an adhesive polymer include polyvinyl alcohol, polyvinyl acetate, polyethylene glycol, polyacrylamide, starch and carboxymethylcellulose, and is preferably biocompatible. A concentration of the adhesive polymer in the microfiber-containing dispersion is preferably, but not limited to, 0.01 to 2 % by weight, more preferably 0.05 to 1 % by weight, further preferably 0.1 to 0.8 % by weight.

The second production method will be described. In the second production method, it is again preferable to carry out diamine modification on the microfibers having carboxy groups as in the first production method, and it is preferable to produce a thin sheet such that the microfibers are connected to each other to form a porous structure before diamine modification. Thus, the microfibers constituting the thin sheet have an amino group. A method for producing the thin sheet and a diamine-modification method are preferably as described for the first production method.

In the light of a smooth dehydration-condensation reaction in the diamine modification, a preferred embodiment is that the thin sheet is treated with acid before initiating diamine modification. A method of acid treatment is preferably as described for the first production method.

Next, the organic dye compound is reacted with the diamine-modified microfibers to be fixed, providing an artificial retina of the present invention. The organic dye compound is reacted preferably as described in the first production method.

In an artificial retina of the present invention, an organic dye compound capable of inducing a receptor potential responding to photostimulation is fixed. As shown in Patent No. 5090431, Matsuo, one of the present inventors, et al. has found that using an organic dye compound capable of inducing a receptor potential responding to photostimulation, a prominent receptor potential is observed as an intracellular potential in optic nerve, particularly in retinal neuronal cells constituting optic nerve. It, therefore, indicates that a film in which an organic dye compound capable of inducing a receptor potential responding to photostimulation is fixed is useful as an artificial retina. In particular, the artificial retina of the present invention is characterized by having liquid permeability due to a porous structure in which the microfibers are connected to each other, and compared to conventional artificial retinas that are not liquid-permeable, it allows body fluids, blood and the like to pass through, resulting in improved biocompatibility. Thus, deterioration of a retinal tissue can be suppressed when the artificial retina of the present invention is in close contact with the retinal tissue. Therefore, a preferred embodiment is that it is used in close contact with a retinal tissue, and it is used as an artificial retina that is very useful in both clinical trials and nonclinical trials.

## Examples

The present invention will be further specifically described with reference to Examples. In these examples, an

organic dye compound used was a polymethine type organic dye compound represented by the formula shown below, which was reported and produced as "NK-5962" from Hayashibara Co., Ltd.

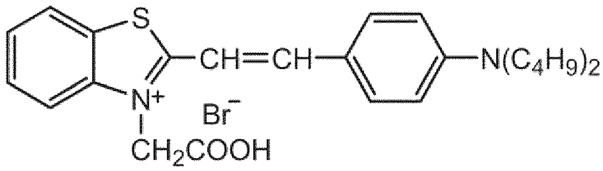

Example 1

[Preparation of a TOCN porous material]

Distilled water was added to RHEOCRYSTA (DKS Co. Ltd., solid content: 2.0 wt%, average fiber diameter: 4 nm) which is a cellulose nanofiber having carboxyl groups (TOCN: TEMPO-oxidized CNF) to a TOCN concentration of 1.0 wt%, and was ultrasonic-irradiated for 1 hour using a tabletop type ultrasonic cleaner (BRANSON, 2510J-MT) to prepare an aqueous TOCN dispersion. After defoaming for 30 minutes using a planetary centrifugal mixer (THINKY, AR-100), the dispersion was transferred to a stainless steel petri dish and allowed to stand in a freezer overnight. The dispersion was subjected to freeze-drying using a freeze dryer (EYELA, FDU-1200) to provide a TOCN porous material. FIG. 1 shows a photograph of the obtained TOCN porous material. A porosity calculated using the following equation was 99%, and it was confirmed that the TOCN has a high specific surface area.

$$\text{Porosity (\%)} = \left\{1 - \frac{\text{Density of porous material}}{1.5\ (= \text{density of CNF})}\right\} \times 100$$

[Diamine modification]

In a 200 mL stoppered Erlenmeyer flask were charged 0.1 g of TOCN porous material, broken into pieces of about 5 mm with a pair of tweezers, 2.6 μL ($4.0 \times 10^{-5}$ mol) of ethylenediamine, 75 mL of chlorobenzene as a solvent, and $8.25 \times 10^{-3}$ g ($4.0 \times 10^{-5}$ mol) of N,N'-dicyclohexylcarbodiimide (DCC) as a dehydration condensation agent. The flask was placed in a constant-temperature water bath shaker (EYELA, NTS-4000AM) at 35 °C and the mixture was reacted with stirring at 50 rpm for 48 hours. The TOCN porous material to which ethylenediamine was attached (hereinafter, referred to as "diamine-modified porous material") was taken out, and washed with chlorobenzene by filtration. The diamine-modified porous material was used in the next process of dye fixing without being dried.

[Dye fixing]

In a 200 mL stoppered Erlenmeyer flask were charged $2.0 \times 10^{-2}$ g ($4.0 \times 10^{-5}$ mol) of a photoelectric conversion dye (Hayashibara Co., Ltd., NK-5962), 75 mL of chlorobenzene as a solvent, and $8.25 \times 10^{-3}$ g ($4.0 \times 10^{-5}$ mol) of DCC as a dehydration condensation agent. The dye was dispersed by ultrasonic irradiation for 30 minutes using a tabletop ultrasonic cleaner. Next, the diamine-modified porous material was added, and the mixture was reacted for 48 hours with stirring at 50 rpm in a constant-temperature water bath shaker at 35 °C. The TOCN porous material to which the photoelectric conversion dye has been attached (hereinafter, referred to as "dye-fixed porous material") was taken out, washed with chlorobenzene by filtration, and naturally dried.

[Preparation of a dye-fixed thin sheet]

In a 100 mL eggplant flask was weighed 0.1 g of PVA powder (Sigma-Aldrich, saponification degree: 99 % or more, average molecular weight: 89,000 to 98,000), and distilled water was added to the mixture such that a PVA concentration was 0.5 wt%, and the mixture was completely dissolved in a 95 °C oil bath under reflux, and then allowed to be cooled to room temperature to prepare an aqueous PVA solution. In a 100 mL beaker was added 0.1 g of dye-fixed porous material and the aqueous PVA solution (concentration: 0.5 wt%) and then distilled water was added to the total volume of 50 mL. The mixture was ultrasonic-irradiated for 1 hour using a tabletop ultrasonic cleaner, to prepare an aqueous dispersion of TOCN : PVA = 1 : 1 by weight. The dispersion was transferred to a PFA petri dish, dried at 40 °C, washed with methanol, and naturally dried to prepare a dye-fixed thin sheet. A thickness of the dye-fixed thin sheet thus obtained was about 30 μm. FIG. 2 shows a photograph of the dye-fixed thin sheet obtained.

[Elemental analysis by energy dispersive X-ray analysis (EDX)]

In order to confirm that the dye was chemically fixed on the dye-fixed thin sheet, elemental analysis of the dye-fixed thin sheet was performed using a scanning electron microscope (JEOL, JSM-IT100) equipped with an energy dispersive X-ray spectrometer (EDX). The acceleration voltage was 5 keV and an irradiation current was 60 probe current (PC). In addition, Au was vapor-deposited onto the surface of the sample using an ion coater (EIKO Corporation, IB-3) before SEM observation. FIG. 3 shows an EDX spectrum. On the surface of the dye-fixed thin sheet, a large peak of Na derived from the COONa-type carboxy group, a peak of Br derived from the dye, and small peaks of N derived from the dye and amide bonds were observed. Thus, it was confirmed that the dye was chemically fixed on the dye-fixed thin sheet.

[Evaluation of liquid permeability]

To evaluate the liquid permeability, the dye-fixed thin sheet was sandwiched between filter papers, phosphate-buffered saline (PBS, pH 7.2) was added dropwise from one side, and the weight of the filter paper on the other side was measured. As a result, the weight of the filter paper before dropping was 0.0140 g, while the weight of the filter paper after dropping was 0.0252 g. Since the weight of the filter paper on the other side increased, it was confirmed that the liquid penetrated through. Therefore, it was found that the dye-fixed thin sheet is liquid-permeable.

[Measurement of photoinduced surface potential]

To confirm that the dye-fixed thin sheet produced a potential when exposed to light, a scanning Kelvin probe (KP Technology, SKP5050) was used to measure a photoinduced surface potential. The device was placed in an atmospheric pressure replacement type desiccator. A light intensity was controlled using a surface photovoltage spectroscopy (SPS) module (KP Technology, SPS040), and the measurement was performed in SPV (Surface Photo Voltage) mode in which an intensity of the irradiation light was varied. Light was irradiated with a light intensity being increased by 100 arbitrary units (arb. units) in the range of 0 to 5000 arb. units, and surface potential variation was measured. The measurement results are shown in FIG. 4.

Example 2

[Preparation of a TOCN-COOH thin sheet]

Distilled water was added to RHEOCRYSTA (DKS Co. Ltd., solid content: 2.0 wt%, average fiber diameter: 4 nm) which is a cellulose nanofiber having carboxyl groups (TOCN: TEMPO-oxidized CNF) to a TOCN concentration of 0.2 wt%, and was treated with a homogenizer for 1 hour. Subsequently, the mixture was ultrasonic-irradiated for 1 hour using a tabletop type ultrasonic cleaner to prepare an aqueous TOCN dispersion. The TOCN dispersion was transferred to a PFA petri dish, dried at 50 °C to prepare a TOCN thin sheet. The TOCN thin sheet was cut into a 1.5 cm $\times$ 1.5 cm piece, and the TOCN thin sheet was then allowed to stand in 0.01M hydrochloric acid for 30 min for converting carboxy groups from COONa type to COOH type. The piece was washed with distilled water until the washing became neutral and naturally dried to prepare a TOCN-COOH thin sheet. In order to confirm that carboxy group in the TOCN-COOH thin sheet was converted into COOH type, the TOCN thin sheet and the TOCN-COOH thin sheet were analyzed by infrared spectroscopy (IR) using a Fourier transform infrared spectrophotometer (Shimadzu Co., Ltd., IRAffinity-1S). The IR spectrum obtained is shown in FIG. 5.

[Diamine modification]

In a 200 mL stoppered Erlenmeyer flask were charged the TOCN-COOH thin sheet, 2.6 $\mu$L ($4.0 \times 10^{-5}$ mol) of ethylenediamine, 75 mL of chlorobenzene as a solvent, and $8.25 \times 10^{-3}$ g ($4.0 \times 10^{-5}$ mol) of N,N'-dicyclohexylcarbodiimide (DCC) as a dehydration condensation agent. The flask was placed in a constant-temperature water bath shaker (EYELA, NTS-4000AM) at 35 °C and the mixture was reacted with stirring at 50 rpm for 48 hours. A thin sheet to which ethylenediamine was attached (hereinafter, referred to as "diamine-modified TOCN-COOH thin sheet") was taken out, and washed with chlorobenzene. The diamine-modified TOCN-COOH thin sheet was used in the next process of dye fixing without being dried.

[Dye fixing]

In a 200 mL stoppered Erlenmeyer flask were charged $2.0 \times 10^{-2}$ g ($4.0 \times 10^{-5}$ mol) of a photoelectric conversion dye (Hayashibara Co., Ltd., NK-5962), 75 mL of chlorobenzene as a solvent, and $8.25 \times 10^{-3}$ g ($4.0 \times 10^{-5}$ mol) of DCC as a

dehydration condensation agent. The dye was dispersed by ultrasonic irradiation for 30 minutes using a tabletop ultrasonic cleaner. Next, the diamine-modified TOCN-COOH thin sheet was added, and the mixture was reacted for 48 hours with stirring at 50 rpm in a constant-temperature water bath shaker at 35 °C. A thin sheet to which the photoelectric conversion dye was attached (hereinafter, referred to as "dye-fixed TOCN-COOH thin sheet ") was taken out, washed with chlorobenzene, and then naturally dried. The thin sheet was washed with chlorobenzene, and the color of chlorobenzene was checked every hour after washing. If chlorobenzene was colored, it was washed again for 1 hour. If it was not colored, the washing was completed and the thin sheet was naturally dried. A thickness of the dye-fixed TOCN-COOH thin sheet obtained was about 30 μm. A photograph of the dye-fixed TOCN-COOH thin sheet obtained is shown in FIG. 6.

[Elemental analysis by energy dispersive X-ray analysis (EDX)]

As described in Example 1, elemental analysis of the dye-fixed TOCN-COOH thin sheet was conducted. FIG. 7 shows an EDX spectrum. On the surface of the dye-fixed TOCN-COOH thin sheet, no peaks of Na derived from the COONa type carboxy group were observed, and a large peak of Br, large peaks of N derived from the dye and amide bonds were observed. Thus, it was confirmed that the dye was chemically fixed on the dye-fixed TOCN-COOH thin sheet. Furthermore, compared to the elemental analysis results of the dye-fixed thin sheet in Example 1, the Br and N peaks were observed to be larger, suggesting that more dye was chemically fixed.

[Evaluation of liquid permeability]

As described in Example 1, liquid permeability was evaluated. As a result, the weight of the filter paper before the drop was 0.0135 g, while the weight of the filter paper after dropping was 0.0288 g. Since the weight of the filter paper on the opposite side increased, it was confirmed that the liquid penetrated through. Therefore, it was found that the dye-fixed TOCN-COOH thin sheet is liquid-permeable. After absorbing saline, the dye-fixed TOCN-COOH thin sheet maintained its thin film shape without dispersing, and can be used in vivo without being broken.

[Measurement of photoinduced surface potential]

As described in Example 1, a photoinduced surface potential of the dye-fixed TOCN-COOH thin sheet was measured. FIG. 8 shows the measurement results obtained.

[Ultraviolet-visible spectrometry]

An absorbance of the dye-fixed TOCN-COOH thin sheet prepared in Example 2 was measured using an ultraviolet-visible spectrophotometer (JASCO Corporation, V-750) equipped with an integrating sphere unit (JASCO Corporation, PIV-75). FIG. 9 shows the results of ultraviolet-visible spectrometry. An absorbance at the peak top of the dye-fixed TOCN-COOH thin sheet was 1.104. On the other hand, as a comparison, an absorbance of a thin film of polyethylene on which a dye was fixed was measured and was about 0.1. Thus, it can be seen that the amount of dye fixed increased.

## Claims

**1.** An artificial retina in which an organic dye compound is fixed onto a thin sheet, wherein

the organic dye compound induces a receptor potential responding to photostimulation;
the thin sheet is formed by connecting microfibers having an average fiber diameter of 2 to 1,000 nm to each other to form a porous structure; and
the porous structure makes the artificial retina liquid-permeable.

**2.** The artificial retina according to Claim 1, wherein the organic dye compound is fixed onto the microfibers in the thin sheet via a covalent bond.

**3.** The artificial retina according to Claim 1 or 2, wherein the microfibers are biocompatible.

**4.** A method for producing the artificial retina according to any one of Claims 1 to 3, comprising

diamine-modifying the microfibers having carboxy groups;

reacting the organic dye compound with the diamine-modified microfibers to fix the organic dye compound to the microfibers; and then
producing the thin sheet such that the microfibers are connected to each other to form a porous structure.

**5.** A method for producing the artificial retina according to any one of Claims 1 to 3, comprising

producing the thin sheet such that the microfibers are connected to each other to form a porous structure; then
diamine-modifying the microfibers which have carboxy groups and constitute the thin sheet; and
reacting the organic dye compound with the diamine-modified microfibers constituting the thin sheet to fix the organic dye compound to the microfibers.

[FIG. 1]

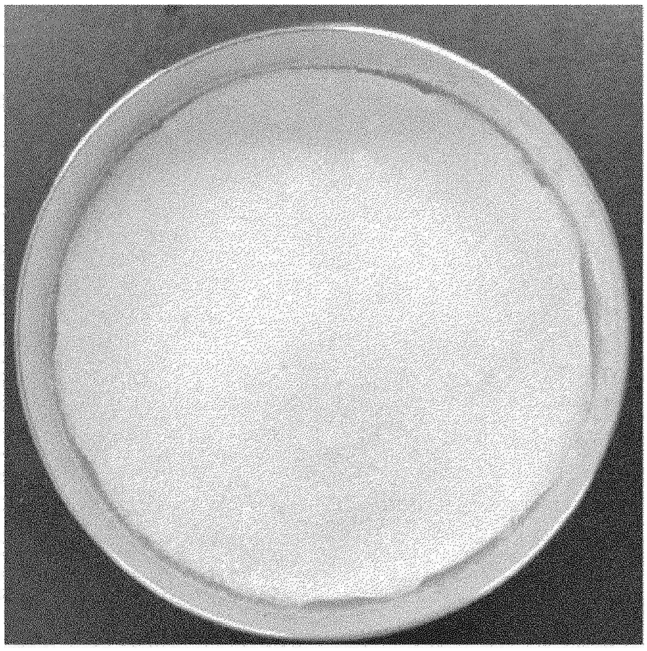

[FIG. 2]

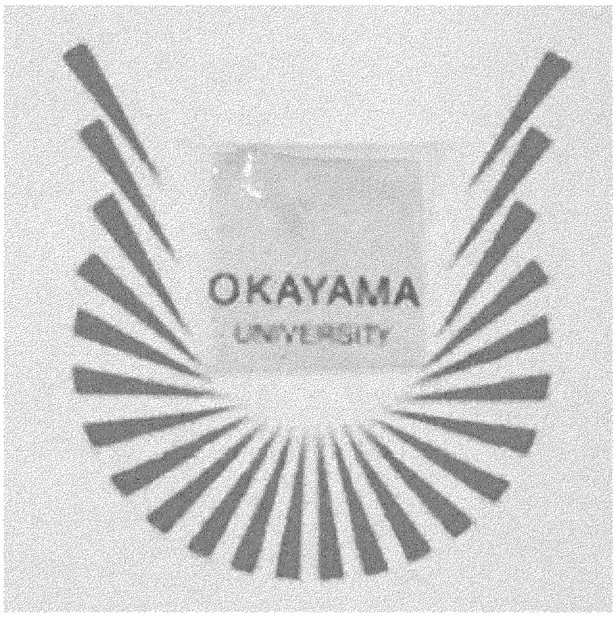

[FIG. 3]

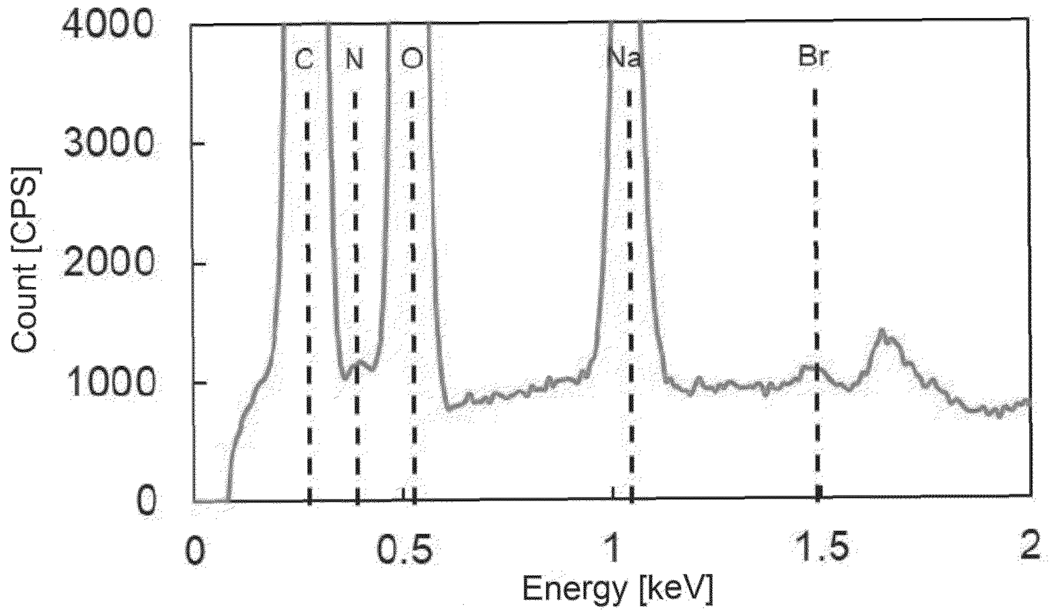
4000
3000
2000
1000
0
Count [CPS]
C
N
O
Na
Br
0
0.5
1
1.5
2
Energy [keV]

[FIG. 4]

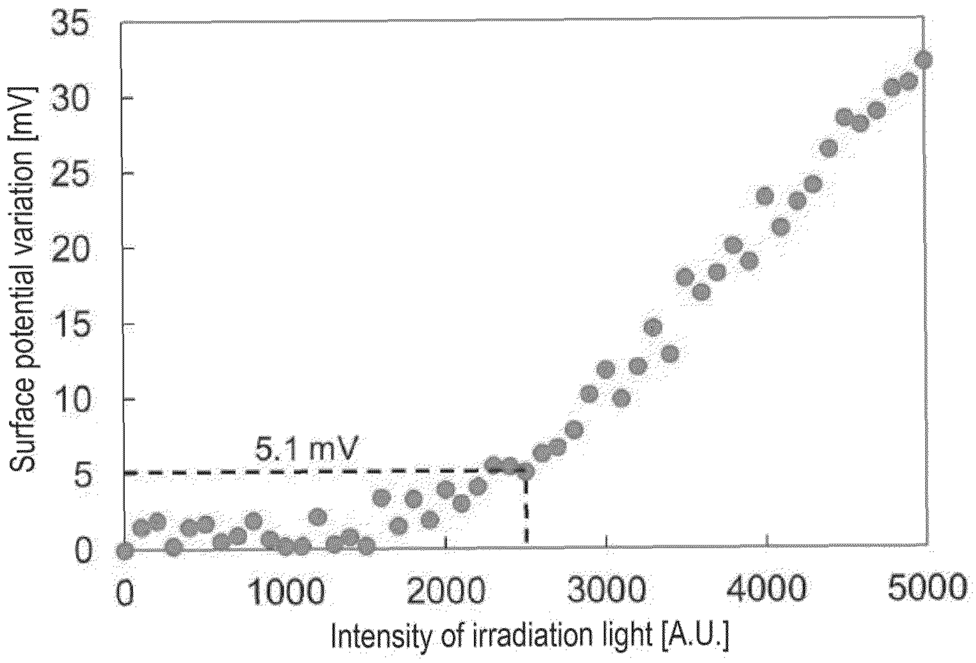
35
30
25
20
15
10
5
0
Surface potential variation [mV]
5.1 mV
0
1000
2000
3000
4000
5000
Intensity of irradiation light [A.U.]

[FIG. 5]

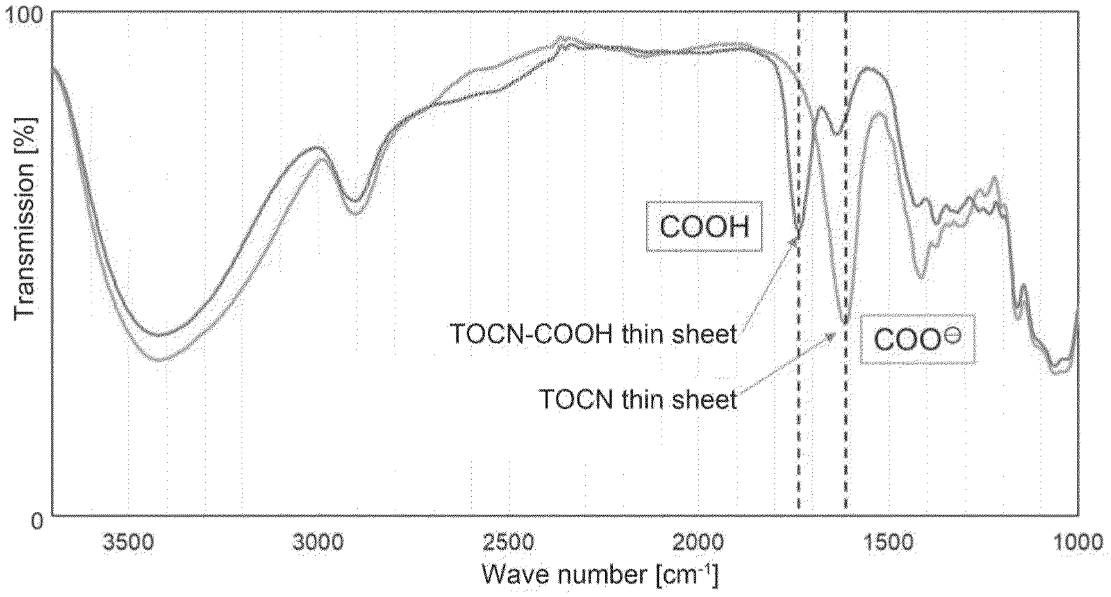
100
0
Transmission [%]
3500
3000
2500
2000
1500
1000
Wave number [cm-1]
COOH
COO⊖
TOCN-COOH thin sheet
TOCN thin sheet

[FIG. 6]

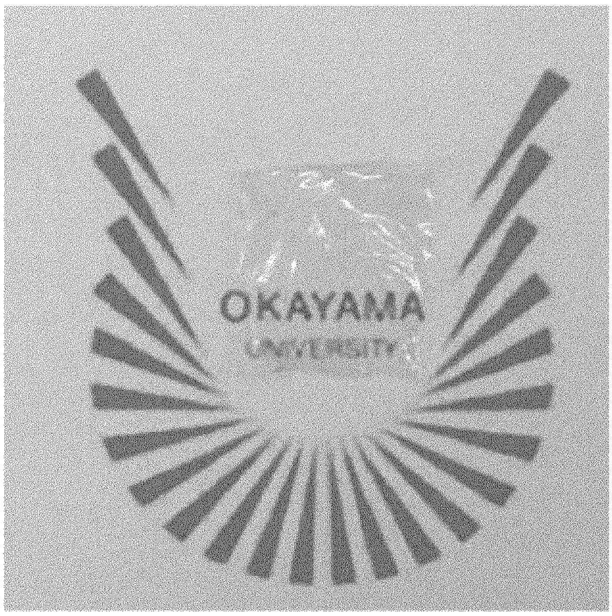
OKAYAMA
UNIVERSITY

[FIG. 7]

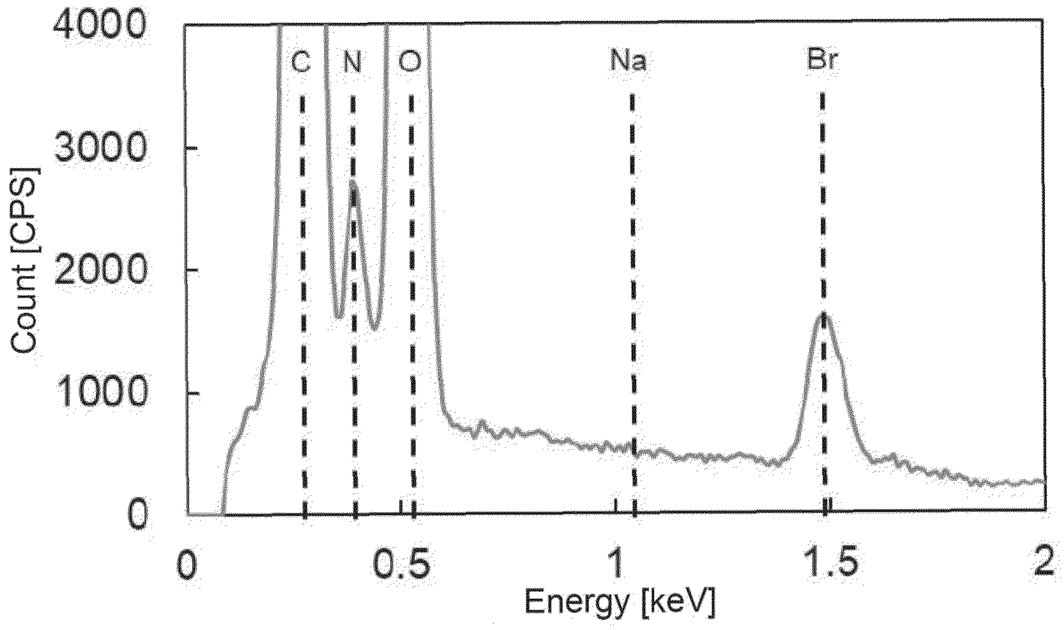
4000
3000
2000
1000
0
Count [CPS]
C
N
O
Na
Br
0
0.5
1
1.5
2
Energy [keV]

[FIG. 8]

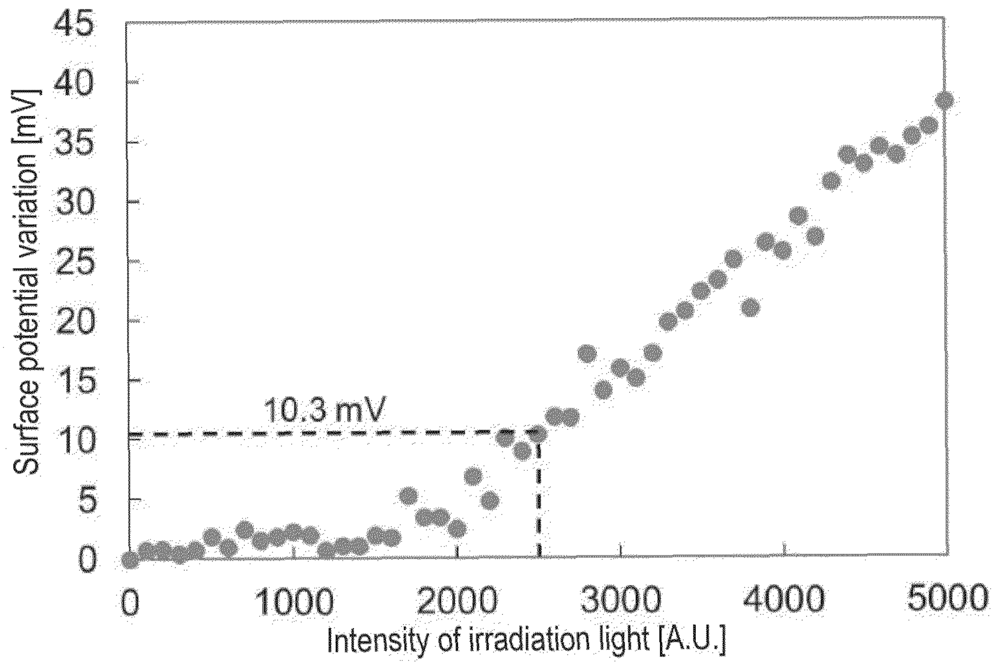
45
40
35
30
25
20
15
10
5
0
Surface potential variation [mV]
10.3 mV
0
1000
2000
3000
4000
5000
Intensity of irradiation light [A.U.]

[FIG. 9]

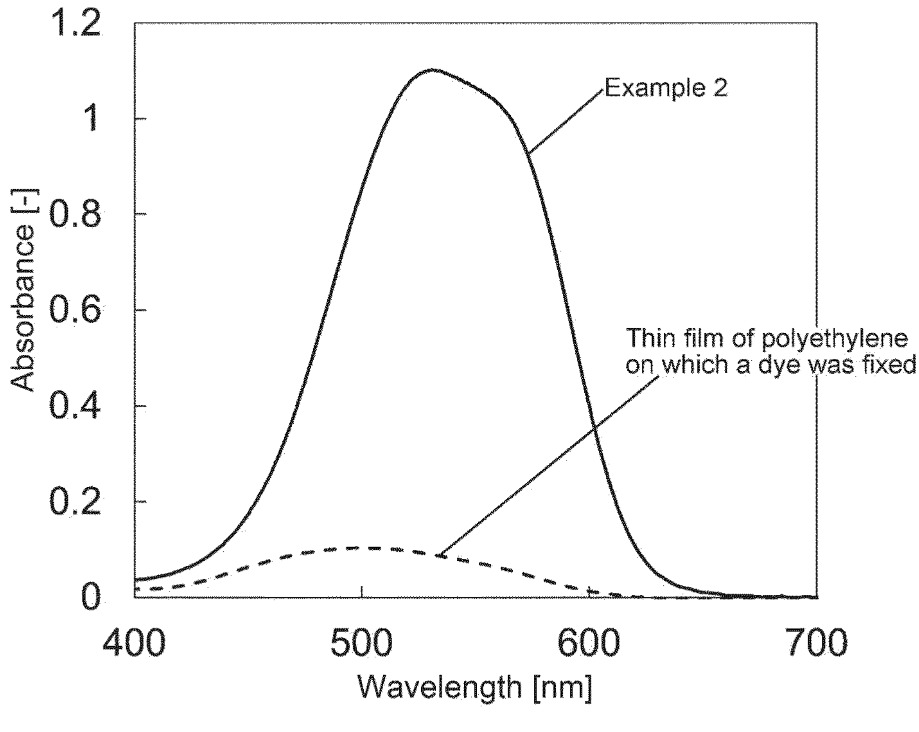
1.2
1
0.8
0.6
0.4
0.2
0
Absorbance [-]
400
500
600
700
Wavelength [nm]
Example 2
Thin film of polyethylene
on which a dye was fixed

**INTERNATIONAL SEARCH REPORT**

International application No.
**PCT/JP2023/009870**

**A. CLASSIFICATION OF SUBJECT MATTER**

***A61F 9/007***(2006.01)i; ***A61L 27/14***(2006.01)i; ***A61L 27/50***(2006.01)i
FI: A61F9/007 190B; A61L27/14; A61L27/50

According to International Patent Classification (IPC) or to both national classification and IPC

**B. FIELDS SEARCHED**

Minimum documentation searched (classification system followed by classification symbols)

A61F9/007; A61L27/14; A61L27/50; A61F2/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br>A | WO 2015/152233 A1 (NATIONAL UNIVERSITY CORPORATION OKAYAMA UNIV.) 08 October 2015 (2015-10-08)<br>paragraphs [0013]-[0025] | 1-3<br>4-5 |
| Y<br>A | US 7364674 B1 (ADVANCED OPTICAL TECHNOLOGIES, INC.) 29 April 2008 (2008-04-29)<br>column 4, line 66 to column 5, line 31., fig. 1 | 1-3<br>4-5 |
| A | JP 2004-121292 A (HAYASHIBARA BIOCHEM. LAB., INC.) 22 April 2004 (2004-04-22)<br>entire text, all drawings | 1-5 |
| A | ALAMUSI et al., Behavior tests and immunohistochemical retinal response analyses in RCS rats with subretinal implant, Journal of Artificial Organs, 26 March 2013<br>entire text, all drawings | 1-5 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 May 2023** | **30 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/009870**

**C. DOCUMENTS CONSIDERED TO BE RELEVANT**

| Category* | Citation of document, with indication, where appropriate, of the relevant passages | Relevant to claim No. |
|---|---|---|
| A | MATSUO, Toshihiko et al. Safety, efficacy, and quality control of a photoelectric dye-based retinal prosthesis (Okayama University-type retinal prosthesis) as a medical device. OKAYAMA UNIV. Journal of Artificial Organs. 25 December 2009.<br>entire text, all drawings | 1-5 |
| A | 松尾俊彦, 医療と画像処理 岡山大学方式の人工網膜の試作品 光電変換色素をポリエチレン・フィルムに固定した人工網, 画像ラボ, 01 September 2006, (Image Laboratory), non-official translation (MATSUO, Toshihiko. Medical and Imaging, Prototype of artificial retina of Okayama University method, An artificial net in which photoelectric conversion dyes are fixed on a polyethylene film.)<br>entire text, all drawings | 1-5 |

Form PCT/ISA/210 (second sheet) (January 2015)

**INTERNATIONAL SEARCH REPORT**
**Information on patent family members**

International application No.
**PCT/JP2023/009870**

| Patent document cited in search report | Publication date (day/month/year) | Patent family member(s) | Publication date (day/month/year) |
|---|---|---|---|
| WO 2015/152233 A1 | 08 October 2015 | US 2017/0106121 A1<br>paragraphs [0018]-[0030]<br>EP 3127558 A1 | |
| US 7364674 B1 | 29 April 2008 | (Family: none) | |
| JP 2004-121292 A | 22 April 2004 | US 2004/0062713 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004121292 A
- WO 2015152233 A1
- JP 5090431 B
- JP 2020146296 A
- WO 5090431 A, Matsuo